# EUROPEAN PATENT APPLICATION

(11) **EP 4 813 448 A1**
(43) Date of publication of application: **30.09.2026**
(21) Application number: 25166589.9
(22) Date of filing: 27.03.2025
(51) Int. Cl.: A61N 5/10

(54) **GENERATING TREATMENT PLANNING OBJECTIVE FUNCTIONS USING COST GRADIENTS**

(71) Applicant: Siemens Healthineers International AG, 6312 Steinhausen (CH)
(72) Inventor: KUUSELA, Esa, 02320 Espoo (FI)
(74) Representative: Mathisen & Macara LLP

(57) **Abstract**

A radiotherapy treatment plan generation apparatus configured to: (i) receive a treatment planning objective function comprising a plurality of clinical goals and plan metrics; (ii) display one or more graphical gradient indicators, each of the graphical gradient indicators corresponding to a plan metric; wherein a visual characteristic of each of the graphical gradient indicators is related to a rate of change of an objective function value with respect to the plan metric; (iii) receive input to perturb the objective function based on the displayed one or more graphical gradient indicators; (iv) update the visual characteristics of the displayed graphical gradient indicators with the rates of change of a value of the perturbed objective function with respect to the plan metrics; (v) repeat steps (iii)-(iv) until an iteration stop criterion is met; when the iteration stop criterion is met, output the perturbed objective function.

## Description

### TECHNCAL FIELD

This invention relates to radiotherapy treatment planning, and in particular, to methods and systems for generating and improving objective functions for use in optimising radiotherapy treatment plans.

### BACKGROUND

### Radiotherapy

The use of energy to treat medical conditions comprises a known area of prior art endeavour. For example, radiation therapy comprises an important component of many treatment plans for reducing or eliminating unwanted tumours. Unfortunately, applied energy does not inherently discriminate between unwanted material and adjacent tissues, organs, or the like that are desired or even critical to continued survival of the patient (such adjacent tissues may be referred to as "organs at risk", OARs). As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform "machine" parameters during each of a plurality of sequential fields. For example, machine parameters of a radiotherapy system may include the angle of irradiation of the treatment beam, or the configuration of the leaves of a multileaf collimator.

### Plan optimisation using cost functions

Treatment plans for radiation treatment sessions are often automatically generated through a so-called optimisation process. As used herein, "optimisation" will be understood to refer to improving a candidate treatment plan without necessarily ensuring that the optimised result is, in fact, the singular best solution. Such optimisation often includes automatically adjusting one or more physical treatment machine parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

In some cases, optimisation proceeds as a function of multiple different criteria. This may involve the use of a cost function comprising a mathematical model that attempts to balance conflicting clinical goals to produce a treatment plan with the least "cost". Clinical goals generally take the form of a particular metric being required to be greater or smaller than a threshold value. For example, a clinical goal may require that a certain percentage of the volume of an OAR receives less than a certain amount of dose. As a further example, a clinical goal may require that a certain percentage of the planning target volume (PTV) receives at least a certain amount of dose. A cost function may be a sum of terms relating to deviations from the clinical goals. Generally, a cost function may be any function that quantifies the deviation of treatment plan metrics from corresponding clinical goals.

As an alternative to a cost function, a utility function may be used. Utility functions have terms that reward adherence to clinical goals, wherein said optimisation comprises maximising the utility function. In general, in the present disclosure, a "utility function" may be used instead of a "cost function", wherein the optimiser attempts to maximise the utility of the utility function instead of minimising the cost of the cost function.

A computer-implemented optimiser is generally used to iteratively maximise (or minimise) the utility function (or cost function). In general, the process of optimisation involves finding a set of machine parameters (also referred to as "control points") that maximise a given utility function (or minimise the cost function).

### Determining the objective function

As noted, in radiotherapy treatment planning, an objective function as described above is first determined, and then a plan is generated by optimising the objective function - for example, by minimising a cost function or maximising a utility function. While the use of an objective function in optimising a treatment plan is well known in the prior art, the generation of the objective function itself needs further exploration.

In prior art schemes, a human planner may construct an objective function such as a cost function by defining a set of objectives. Examples of objectives include a mean dose objective for a tumour, a normal tissue dose objective or a fluence smoothing objective. In selecting the objectives, the human planner may be guided by clinical knowledge and practical experience of how the selection of different combinations of plan objectives leads to desired clinical outcomes, such as tumour control, the avoidance of side effects, or the meeting of quality assurance requirements.

Some objectives may be conflicting, in the sense that an improvement in attaining a first objective leads to a deterioration in attaining a conflicting objective. Further, some objectives may be too ambitious while other objectives may be too easy to achieve. For example, if the cost of a cost function is dominated by one of the objectives to the detriment of the others, then this may indicate that the optimiser needs to make significant compromises with the other objectives in attempting to meet the dominating objective in question. Similarly, if the overall cost of a cost function is very little affected by one of the objectives, it may indicate that this particular objective may be made more ambitious. In selecting goals and objectives to determine an objective function for treatment planning, the human planner will need to consider these types of tradeoffs.

It may take the human planner multiple attempts to find a set of treatment plan objectives that provide an optimal treatment plan, and this may involve some degree of trial and error. For example, the human planner may change a goal or objective of the objective function and then see how this changes the metrics associated with a treatment plan optimised using the changed objective function. As another example, after using an objective function to generate and optimise a treatment plan, the human user may wish to know if optimising using a different objective function would have resulted in a superior treatment plan.

A further consideration is the use of objective functions wherein the individual cost terms are not associated with a single goal, or where a single goal is associated with multiple anatomical structures. There is a need in the prior art for computer-assisted methods for the selection of appropriate goals or objectives for such objective functions.

Some prior art techniques may visualise the absolute cost of each term in an objective function comprising a sum of terms. However, these do not cater for objective functions of a more general form, such as forms other than a simple sum of terms.

In summary, rather than being concerned only with the generation or optimisation of a radiotherapy treatment plan using objective functions *per se,* the problem presently addressed is the determination of optimal objective functions themselves, and appropriate selection of aspects of the objective functions, such as clinical goals and objectives, goal weights or goal priorities.

### SUMMARY OF THE INVENTION

In accordance with the invention, there is provided a radiotherapy treatment planning generation apparatus as defined by claim 1.

Optional features are defined by the dependent claims.

In accordance with a first aspect of the invention, there is provided a radiotherapy treatment plan generation apparatus configured to:
(i) receive a treatment planning objective function comprising a plurality of clinical goals and plan metrics;
(ii) display one or more graphical gradient indicators, each of the graphical gradient indicators corresponding to a plan metric; wherein a visual characteristic of each of the graphical gradient indicators is related to a rate of change of an objective function value with respect to the plan metric;
(iii) receive input to perturb the objective function based on the displayed one or more graphical gradient indicators;
(iv) update the visual characteristics of the displayed graphical gradient indicators with the rates of change of a value of the perturbed objective function with respect to the plan metrics;
(v) repeat steps (iii)-(iv) until an iteration stop criterion is met;
when the iteration stop criterion is met, output the perturbed objective function.

Advantageously, the human planner may use the displayed graphical gradient indicators to guide decisions regarding how to perturb the objective function. This is performed iteratively, where the graphical gradient indicators are updated after every perturbation of the objective function. The final objective function outputted at the end of the ongoing human-computer interactive process may provide superior treatment plans compared to plans provided by the initial objective function.

### Gradient bars

Optionally, the graphical gradient indicators each comprise a variable-length shape such as a bar, an ellipse of varying diameter, or a triangle of varying hypotenuse length, and wherein said visual characteristic comprises the length of the variable-length shape.

Advantageously, the length of a variable-length shape such as a bar may represent the rate of change of the overall value of the objective function in response to changes of the corresponding plan metric. Such bars are referred to as "gradient bars", and provide useful information to the planner in deciding how to perturb the objective function.

For example, if the variable-length shape such as a gradient bar shows that the objective function value is very sensitive to changes in a particular plan metric, the planner may decide to make the corresponding clinical goal less ambitious. Conversely, if the variable-length shape such as a gradient bar shows that the objective function value is not very sensitive to changes in a particular plan metric, the planner may make the corresponding clinical goal more ambitious.

Optionally, the length of each variable-length shape, such as a bar, is proportional to the rate of change of the objective function value in response to changes of the plan metric.

Advantageously, by being proportional to the rate of change of the objective function value in response to changes of the plan metric, the length of the variable-length shape, such as a bar, is representative of the sensitivity of the cost function to the plan metric. This insight into the sensitivity of the cost function to each plan metric provides a human planner with useful information to perturb the objective function; such perturbed objective functions may provide superior treatment plans to the initial objective function.

### Inverted units

Optionally, the length of each variable-length shape, such as a bar, is inversely proportional to the rate of change of the objective function value in response to changes of the plan metric.

Inverting the cost gradient in this manner is useful when the variable-length shapes such as the gradient bars are overlaid on a dose distribution, such as a DVH, in order to further assist the human planner in making objective function perturbation decisions (overlaying the gradient bars on DVHs is discussed in detail later).

Similar to a variable-length shape with a length that is proportional to the rate of change of the objective function value in response to changes of the plan metric, by being inversely proportional to the rate of change, the length of the variable-length shape is representative of the sensitivity of the cost function to the plan metric. However, by having lengths inversely proportional to the rate of change rather than proportional to the rate of change, the variable-length shapes or gradient bars corresponding to dose plan metrics may more effectively be overlaid on a dose-volume histogram where the x-axis of the DVH corresponds to dose.

For example, the metric in question may be the Dose-at-Volume of a particular treatment plan; the units of such a metric would be dose. Further, the units of the x-axis of a DVH is dose. When overlaying the variable-length shapes or gradient bars on the DVH, it would be useful if the variable-length shape or the gradient bar had the same units as the DVH, that is, units comprising dose. This would aid the human planner in comparing the variable-length shape or gradient bar against the DVH.

However, the units of the rate of change of the objective function value in response to changes in a dose plan metric such as Dose-at-Volume is 1/dose (the change of the objective function value is a scalar value with no units while the units of the change of the Dose-at-Volume is dose).

Thus, by making the length of the variable-length shape or gradient bar inversely proportional to the rate of change of the objective function value in response to changes in a dose plan metric, the units of the variable-length shape or the gradient bar would be dose (for example, grays), making for easy comparison with the DVH when the gradient bar is overlaid on the DVH. This is not possible when the length of the gradient bar is directly proportional (rather than inversely proportional) to the rate of change of the objective function value in response to changes of the Dose-at-Volume metric.

Inverting the cost gradients of dose-based metrics is not necessary when the graphical gradient indicators are not overlaid on a graph wherein the units of the relevant axis of the graph does not comprise dose.

### Perturbing the objective function

Optionally, perturbing the objective function comprises changing aspects of the objective function, such as clinical goals, clinical goal weights or clinical goal priorities.

In particular, perturbing the objective function may comprise modifying any combination of one or more of the clinical goals, clinical goal weights or clinical goal priorities. Perturbing the objective function may also comprise modifying any combination of plan objectives, plan objective weights, or plan objective priorities.

By iteratively modifying the clinical goals, clinical goal weights or clinical goal priorities based upon the information provided by the graphical gradient indicators, a superior objective function may be obtained that is capable of generating superior radiotherapy treatment plans.

For example, a clinical goal that is deemed to be overly ambitious in view of the graphical gradient indicators may be modified to be less ambitious. An overly ambitious goal may make it harder to achieve other goals that are conflicting with the overly ambitious goal. Alternatively or additionally, the weight of an overly ambitious goal may be reduced, or the corresponding priority may be lowered.

Conversely, a clinical goal that is deemed to be not very ambitious in view of the graphical gradient indicators may be modified to be more ambitious. Such a clinical goal may be modified without overly affecting the extent to which other conflicting goals may be achieved. Alternatively or additionally, the weight of a goal that is found to be not ambitious enough may be increased, or the corresponding priority may be increased.

Similarly, a clinical goal associated with a metric that greatly influences cost can be made less ambitious in order to reduce the cost, and clinical goals associated with metrics that do not greatly influence cost may be made more ambitious without greatly affecting the cost.

The extent to which changes in the metrics change the cost is reflected in the graphical gradient indicators such as the gradient bars. In general, improving the cost associated with meeting one clinical goal may result in deteriorating the cost associated with another, conflicting clinical goal.

Optionally, the apparatus may perturb the objective function based on the length of the variable-length shapes, such as the gradient bars discussed above.

As discussed, the length of the variable-length shapes or the gradient bars provides useful information regarding how to advantageously modify the objective function to ultimately provide better plans, because the variable-length shapes or gradient bars show how sensitive the cost is to changes in various metrics.

In general, either the human planner or the apparatus may perturb the objective function in view of the rates of change of the objective function value in response to changes in the plan metrics.

### Scaling the graphical gradient indicators

Optionally, the apparatus may be configured to scale the graphical gradient indicators with a linear scaling coefficient.

For example, the graphical gradient indicators may be scaled so that they may be better overlaid on a DVH of an OAR, to provide clearer information on the relationship between the variable-length shapes or gradient bars and the dose axis of the DVH. For example, the length of the variable-length shapes or gradient bars may be scaled to correspond to the scale of the dose axis of the DVH.

Optionally, the apparatus may be further configured to use (i) a relatively smaller linear coefficient to visualise rates of change with respect to a current treatment plan solution, and (ii) a relatively larger linear coefficient to visualise differences between the rates of change.

For example, the variable-length shapes or gradient bars may be overlaid on the DVH at the point of the corresponding plan metric. Variable-length shapes or gradient bars that are scaled to be relatively short may help to visualise the solution at the vicinity of the point at which the variable-length shape or gradient bar crosses the DVH. However, this makes it harder to assess the differences between the variable-length shapes or gradient bars. Thus, there is a trade-off between using shorter gradient bars that help visualise the solution at the point at which the gradient bar crosses the DVH, and using longer gradient bars where the differences between the gradient bars are more clearly visible.

Optionally, the apparatus is further configured to scale the graphical gradient indicators with a non-linear scaling coefficient, such as an exponential coefficient, or a logarithmic coefficient.

Any type of coefficient may be used to scale the graphical gradient indicators, in dependence on the nature of the graphical information upon which the graphical gradient indicators are overlaid. Simple linear graphs may require simple linear scaling, whereas other types of graph may usefully employ exponential or logarithmic scaling for easy comparison with the overlaid graph.

### Other display options

Optionally, one or more of the graphical gradient indicators each comprise a coloured object, and wherein said visual characteristic comprises a colour of the object.

For example, the visual characteristic of the graphical gradient indicators that represents the cost function gradient may be an intensity or hue of the colour of a dot, a square, a star, or any other suitable shape.

In general, the graphical gradient indicators may take on any shape, appearance or form, as long as a visibly distinguishable characteristic of the graphical gradient indicator usefully provides an insight into the rate of change of an objective function value with changes to a plan metric.

Optionally, the apparatus may be further configured to display an arrow indicating the direction of change of the plan metric in response to perturbing the objective function.

Advantageously, such an arrow provides information to the human planner with regard to how to change the objective function. If a particular manner of perturbing the objective function will result in a desirable change to the plan metric achieved by the treatment plan, then the human planner may be encouraged to perturb the objective function accordingly. A desirable change to the plan metric may be a change that brings the plan metric closer to the clinical goal. This desirable change of the plan metric may be depicted by an arrow overlaid, for example, on a DVH of a corresponding organ. For example, if the Dose-to-Volume of an OAR changes favourably when the objective function is perturbed, the direction of this change is depicted by an arrow superimposed on the DVH.

### Overlaying gradient indicators on a dose distribution

Optionally, the apparatus is further configured to display a dose distribution related to the objective function and overlay the graphical gradient indicators on the displayed dose distribution.

For example, the DVH of an OAR may be displayed in the graphical user interface. The desired value of the Dose-to-Volume of the OAR may also be displayed, by for example, a cross located at the required dose/volume point. A graphical gradient indicator indicating the rate of change of the cost in response to changes to the Dose-to-Volume may be superimposed on the DVH at the point of the DVH that represents the achieved Dose-to-Volume. This way, the human planner sees both the DVH and the extent to which the cost will change when the plan's Dose-to-Volume changes, on the same graphical user interface. This aids decision making when perturbing the objective function.

Optionally, the dose distribution comprises a dose-volume histogram.

In principle, any type of dose distribution may be displayed, upon which the graphical gradient indicators may be overlaid, including cumulative dose volume histograms, differential dose volume histograms, dose rate volume histograms, irradiation time volume histograms, and so on.

Optionally, the displayed dose distribution relates to a region of interest, such as an organ at risk or a tumour.

Advantageously, the dose distribution of an organ at risk or a tumour may be easily viewed in conjunction with the graphical gradient indicators. The units of the displayed dose distribution are preferably related to the units of the graphical gradient indicators overlaid on the displayed dose distribution, for easy comparison.

Where the displayed dose distribution is of an organ at risk, a plan metric such as the Dose-to-Volume of the OAR may be superimposed on the dose distribution. Further, the graphical gradient indicators may be superimposed at the point at which the Dose-to-Volume for the OAR intersects the DVH of the OAR. Similarly, where the displayed dose distribution is of a tumour, a plan metric such as the minimum Volume-at-Dose for the tumour may be superimposed on the dose distribution. Graphical gradient indicators may be superimposed at the point at which the Volume-at-Dose for the tumour intersects the DVH of the tumour. The overlaying of all such graphical information on a DVH is described in detail later.

### Exemplary equation for the gradient indicators

Optionally, the rates of change of the objective function value in response to changes to the plan metric comprises a partial derivative such as: $G = \frac{{λ}_{G}}{{\partial C}_{\overline{\partial m}}}$ where:
G = value of the graphical gradient indicator
λ_{G} = scaling coefficient
C = objective function value
m = plan metric

The above equation is merely an example of how the value of the graphical gradient indicator may be calculated and the graphical gradient indicator value may be computed in any number of equation formats.

In the above example, it should be noted that the term that is of most interest is the rate of change of the cost with changes to the metric, i.e., the partial derivative as depicted by $\frac{\partial C}{\partial m}$. However, in this example, the value of the graphical gradient indicator is the inverse of this partial derivative. This is done in order to match the units of the graphical gradient indicators with the units of any dose-volume histogram upon which the graphical gradient indicators may be overlaid.

In particular, a graphical gradient indicator may relate to the rate of change of the cost with respect to changes in a Dose-to-Volume of an OAR. The units of such a graphical gradient indicator is 1/dose. This is because cost is a unit-less scalar whereas the units of Dose-to-Volume is dose. Further, the units of the x-axis of a DVH is dose. Thus, the graphical gradient indicator has units comprising 1/dose, whereas the units of the x-axis of the DVH is dose. For this reason, for easy comparison of the DVH and the graphical gradient indicator, it is useful to invert the value of the graphical gradient indicator; that is, it is useful to use the equation $\frac{{λ}_{G}}{{\partial C}_{\overline{\partial m}}}$ as the value of the graphical gradient indicator (the value of λ_{G} is a scaling coefficient as described above).

As noted above, inverting the value of the graphical gradient indicator is not necessarily required when the graphical gradient indicator is overlaid on a graph that does not use an axis with units that comprise dose.

### Means plus function

In accordance with a second aspect of the invention, there is provided means to generate a treatment planning objective function, comprising means to:
(i) receive a treatment planning objective function comprising a plurality of clinical goals and plan metrics;
(ii) display one or more graphical gradient indicators, each of the graphical gradient indicators corresponding to a plan metric; wherein a visual characteristic of each of the graphical gradient indicators is related to a rate of change of an objective function value with respect to the plan metric;
(iii) receive input to perturb the objective function based on the displayed one or more graphical gradient indicators;
(iv) update the visual characteristics of the displayed graphical gradient indicators with the rates of change of a value of the perturbed objective function with respect to the plan metrics;
(v) repeat steps (iii)-(iv) until an iteration stop criterion is met;
when the iteration stop criterion is met, output the perturbed objective function.

Examples of the means to generate a treatment planning objective function include the computing system and radiation treatment system described below.

### BRIEF DESCRIPTION OF THE DRAWINGS

For a better understanding of the present disclosure, embodiments will now be described by way of example with reference to the accompanying drawings.
Figure 1 shows a block diagram of an example of a computing system upon which some embodiments described herein may be implemented.
Figure 2 is a block diagram showing selected components of a radiation treatment system upon which some embodiments according to the present invention can be implemented.
Figure 3 illustrates elements of an exemplary radiation treatment system upon which radiation treatment plans generated according to some embodiments of the present invention may be implemented.
Figures 4A, 4B and 4C illustrate the rate of change of the cost function value in response to changes in a number of different plan metrics.
Figure 5 shows the overlaying of gradient bars on dose distributions associated with various anatomical structures of interest.
Figure 6 shows steps of an ongoing human-computer interactive process for a radiotherapy treatment plan optimisation process using graphical gradient indicators, in accordance with embodiments of the present invention.

### DETAILED DESCRIPTION

Reference will now be made in detail to several embodiments. While the subject matter will be described in conjunction with the specific embodiments, it will be understood that they are not intended to limit the claimed subject matter to these embodiments. On the contrary, the claimed subject matter is intended to cover alternatives, modifications, and equivalents, which may be included within the scope of the claimed subject matter as defined by the appended claims.

Furthermore, in the following detailed description, numerous specific details are set forth in order to provide a thorough understanding of the claimed subject matter. However, it will be recognised by one skilled in the art that embodiments may be practised without these specific details or with equivalents thereof. In other instances, well-known methods, procedures, components, and circuits have not been described in detail as not to unnecessarily obscure aspects and features of the subject matter.

The following description is presented to enable a person skilled in the art to make and use the embodiments of this invention; it is presented in the context of a particular application and its requirements. Various modifications to the disclosed embodiments will be readily apparent to those skilled in the art, and the general principles defined herein may be applied to other embodiments and applications without departing from the scope of the present disclosure. Thus, the present invention is not limited to the embodiments shown, but is to be accorded the widest scope consistent with the principles and features disclosed herein.

### Computer system

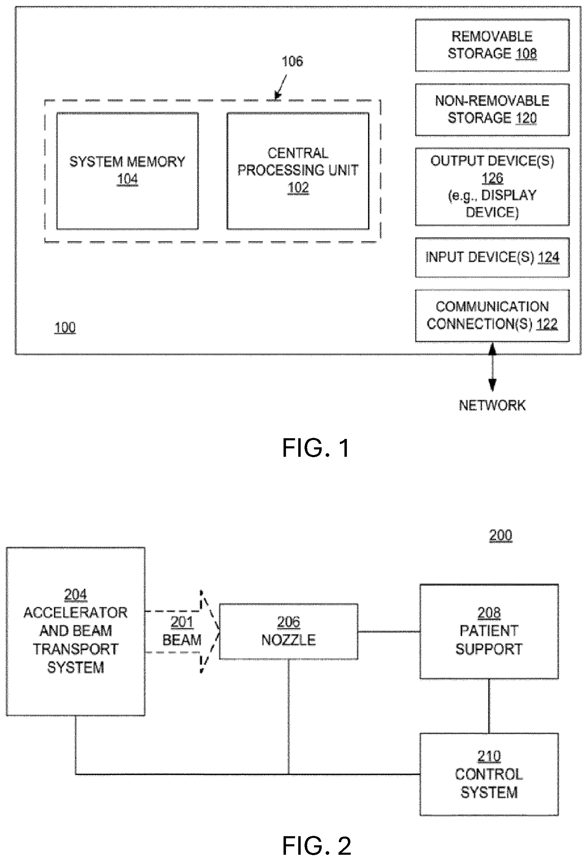

Figure 1 shows a block diagram of an example of a computing system 100 upon which the embodiments described herein may be implemented. In a basic configuration, the system 100 includes at least one processing unit 102 and memory 104. This most basic configuration is illustrated in Figure 1 by dashed line 106. The system 100 may also have additional optional features and/or functionality. For example, the system 100 may also include additional storage (removable and/or non-removable) including, but not limited to, solid state, magnetic or optical disks or tape. Such additional storage is illustrated in Figure 1 by removable storage 108 and non-removable storage 120. The system 100 may also contain communications connection(s) 122 that allow the device to communicate with other devices, e.g., in a networked environment using logical connections to one or more remote computers.

The system 100 may include input device(s) 124 such as keyboard, mouse, pen, voice input device, touch input device, etc. Output device(s) 126 such as a display device, speakers, printer, etc., are also included.

In the example of Figure 1, the memory 104 includes computer-readable instructions, data structures, program modules, and the like. Depending on how it is to be used, the system 100 - by executing the appropriate instructions or the like - can be used to implement a method for generating and improving a radiotherapy treatment planning objective function, as described herein.

### Radiation treatment system

Figure 2 is a block diagram showing selected components of a radiation treatment system 200 upon which embodiments according to the present invention can be implemented. In the example of Figure 2, the system 200 includes an accelerator and beam transport system 204 that is operable to generate and/or accelerate a beam 201. The accelerator and beam transport system can generate and deliver beams of various types including, for instance, X-ray (photon) beams, proton beams, electron beams, ion beams, or atomic nuclei beams (e.g., using elements such as carbon, helium, or lithium). The operations and parameters of the accelerator and beam transport system 204 are controlled so that the intensity, energy, size, and/or shape of the beam are dynamically modulated or controlled during treatment of a patient according to an optimised radiation treatment plan produced by and stored within system 100.

The nozzle 206 is used to aim the beam toward various locations (e.g., of a target) within a patient supported on the patient support device 208 (e.g., a chair, couch, or table) in a treatment room. A target may be an organ, a portion of an organ (e.g., a volume or region within the organ), a tumour, diseased tissue, or a patient outline, for instance.

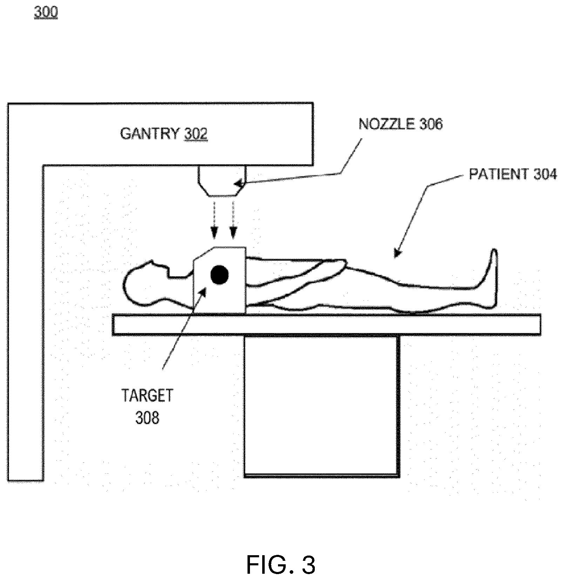

The nozzle 206 may be mounted on or may be a part of a gantry structure (Figure 3) that can be moved relative to the patient support device 208, which may also be moveable. The accelerator and beam transport system 204 may be mounted on or are a part of the gantry structure; alternatively, the accelerator and beam transport system are separate from (but in communication with) the gantry structure.

The control system 210 of Figure 2 receives and implements a prescribed treatment plan which may be generated and/or optimised using the perturbed objective function of the present invention. The control system 210 includes a computing system having a processor, memory, an input device (e.g., a keyboard), and optionally a display; the system 100 of Figure 1 is an example of such a platform for the control system 210. The control system 210 can receive data regarding the operation of the system 200. The control system 210 can control parameters of the accelerator and beam transport system 204, nozzle 206, and patient support device 208, including parameters such as the energy, intensity, size, and/or shape of the beam, direction of the nozzle, and position of the patient support device (and the patient) relative to the nozzle, according to data the control system 210 receives and according to the radiation treatment plan.

Figure 3 illustrates exemplary elements of a radiation treatment system 300 for treating a patient 304. The system 300 is an example of an implementation of the radiation treatment system 200 of Figure 2. The gantry 302 may rotate around an axis, e.g. the gantry may rotate around a patient on a couch. The patient may be moved by moving the couch. While the patient 304 is supine in the example of Figure 3, the invention is not so limited. For example, the patient 304 can instead be seated in a chair or positioned in any orientation. The gantry 302 can be controlled by a treatment system using an optimised treatment plan generated according to the perturbed objective function of the present invention.

### Determining cost functions using gradient bars

The following description discusses the iterative generation and development of cost functions, but it should be understood that a similar method may be performed with any type of objective function, such as utility functions.

The present disclosure relates to an ongoing human-computer interactive process for iteratively determining a radiotherapy treatment planning cost function, wherein the cost function is to be ultimately used in generating and optimising a radiotherapy treatment plan.

A cost function generally comprises a set of clinical goals, wherein each goal is associated with a plan metric. As an example, a plan metric may reflect a treatment plan's dose for an organ at risk, whereas a clinical goal may reflect the desired value of the dose to the organ risk. Similarly, a plan metric may reflect a treatment plan's dose for a tumour, whereas a clinical goal may reflect the desired value of the dose to the tumour. The cost outputted by the cost function is related to a deviation of the plan metrics from the clinical goals. Generally, the greater the deviation of the plan metrics from the clinical goals, the greater the cost associated with a treatment plan. In general, a higher cost indicates a treatment plan with plan metrics that deviate from clinical goals to a greater extent, whereas a lower cost indicates a treatment plan with plan metrics that deviate from clinical goals to a lesser extent.

At the start of the iterative process, an initial cost function is obtained. For example, the initial cost function may be determined by prior art techniques. Then, the rate of change of the cost of the cost function, in relation to changes in the plan metrics, is obtained. In particular, a partial derivative of the cost with respect to changes in an individual plan metric may be obtained. For example, the partial derivative may relate to a rate of change of the cost with a change to the mean dose under the plan to an organ at risk. This partial derivative is referred to as a "cost function gradient".

At the first iteration, one or more graphical gradient indicators may be displayed on-screen, wherein a characteristic of each graphical gradient indicator may be related to a cost function gradient value. For example, a length of a bar displayed on-screen may be proportional to the cost function gradient value, or the colour intensity of a dot displayed on-screen may be proportional to the cost function gradient value.

A human planner may view the graphical gradient indicators that are displayed on-screen in the first iteration. As the graphical gradient indicators show the rate at which the cost changes with respect to changes in metrics, the human planner is provided with information that assists in deciding how to change the cost function to possibly obtain an improved treatment plan. For example, the human planner may use the displayed graphical gradient indicators to change the clinical goals of a cost function.

For example, if the rate of change of the cost with respect to changes of the plan metric "Dose to Volume of OAR #1" is high, then the human planner may choose to change the clinical goal related to the "Dose to Volume of OAR #1".

Changing one or more clinical goals of a cost function will change the extent to which the treatment plan achieves other clinical goals. Making a first clinical goal more ambitious would result in another clinical goal being achieved to a lesser extent. For example, increasing the dose to the tumour would generally result in an increased dose to an organ at risk. Thus, increasing the clinical goal of tumour dose would result in the plan meeting the dose objectives for the organ at risk to a lesser extent. As another example, increasing a priority of a dose objective for a first organ may change the extent to which another dose objective is achieved for a second organ.

If the cost function gradient associated with a first plan metric indicates that the cost is highly sensitive to the first plan metric, but the cost function gradient associated with a second plan metric indicates that the cost is not very sensitive to the second plan metric, the human planner is provided with useful information as to how to perturb or change the cost function to possibly obtain a better treatment plan. For example, the human planner may choose to change clinical goals associated with metrics that greatly influence the cost so that these clinical goals are less ambitious. Additionally or alternatively, the human planner may choose to change clinical goals associated with metrics that do not greatly influence the cost so that these clinical goals are more ambitious.

After the human planner has changed the cost function in the first iteration while being guided by the graphical gradient indicators, the process enters the second iteration. The rates of change of the cost with respect to changes to plan metrics are re-computed for the changed cost function. The graphical gradient indicators are then updated with the changed rates of change of the cost. The human planner may then use the updated graphical gradient indicators to decide how to further perturb the cost function, as in the first iteration.

The process then continues with multiple iterations similar to the second iteration. The cost function is updated or perturbed at each iteration. The iterative process may stop when an iteration stop criterion is met, such as the human planner being satisfied with a treatment plan obtained using the cost function determined by the iterative process. The output of the terminated iterative process is the cost function as perturbed over the multiple iterations. The perturbed cost function may provide a superior treatment plan than the treatment plan provided by the original cost function. Alternatively, the process may comprise only a single iteration. The output of the single-iteration process would be the cost function as perturbed in the single iteration.

### Cost gradients

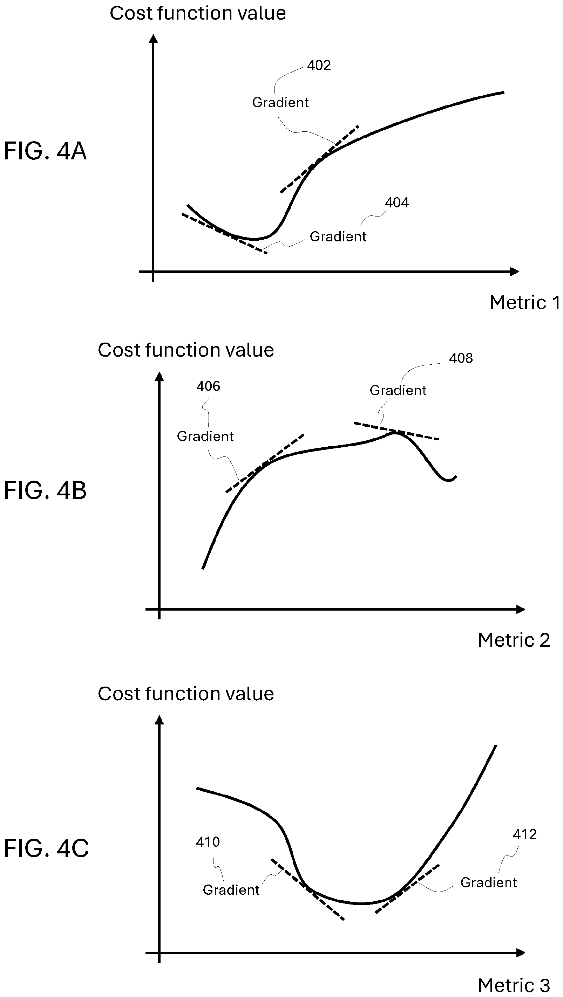

Figures 4A-4C illustrate the notion of a cost function gradient. A cost function gradient for a particular plan metric is the rate of change of the cost of the overall cost function in response to changes of the plan metric. As a cost function is a function of multiple plan metrics but each cost function gradient only relates to a single plan metric, each cost function gradient is referred to as a "partial derivative" of the overall cost with respect to a single plan metric. That is, each plan metric is associated with a cost function gradient.

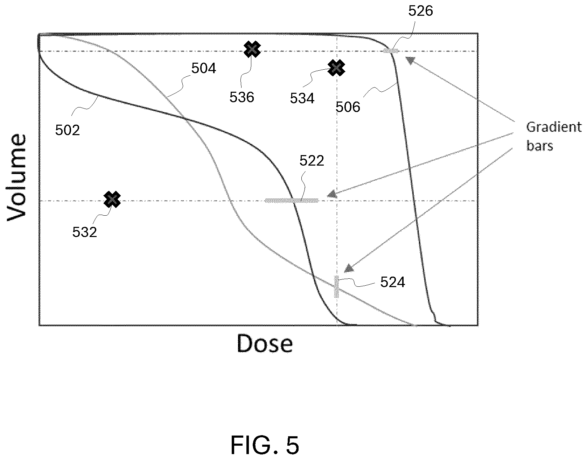

Figure 4A relates to a first metric, which may for example be the mean dose to a tumour. The rate of change of the cost function value with changes to the first metric value are depicted by gradient tangents 402 and 404. Gradient 402 reflects the rate of change of the cost function value when the first metric value is at a certain value, while gradient 404 reflects the rate of change of the cost function value when the first metric value is at another value. The values of these gradients are represented by the graphical gradient indicators of the iterative process discussed above, as will be discussed in relation to Figure 5.

Figure 4B may relate to a second metric, which may for example be the maximum dose to an organ at risk neighbouring the tumour. The rate of change of the cost function value with changes to the second metric value are depicted by gradient tangents 406 and 408. Gradient 406 reflects the rate of change of the cost function value when the second metric value is at a certain value, while gradient 408 reflects the rate of change of the cost function value when the second metric value is at another value. The values of these gradients are represented by the graphical gradient indicators of the iterative process discussed above.

Figure 4C may relate to a third metric similar to the first metric or the second metric, but relating to a different goal such as a minimum dose to the tumour. The rate of change of the cost function value with changes to the third metric value are depicted by gradient tangents 410 and 412. Gradient 410 reflects the rate of change of the cost function value when the third metric value is at a certain value, while gradient 412 reflects the rate of change of the cost function value when the third metric value is at another value. The values of these gradients are represented by the graphical gradient indicators of the iterative process discussed above.

In general, the cost function may comprise any number of metrics similar to the metrics discussed above in relation to Figures 4A-4C.

### Overlaying gradient bars on dose distributions

Figure 5 shows an example of a graphical user interface used in the present ongoing human-computer interactive process for determining a treatment planning objective or cost function.

In particular, the graphical user interface displays a plurality of graphical gradient indicators 522-526 to assist in the ongoing interactive process of perturbing a cost function. Here, the graphical gradient indicators 522-526 comprise horizontal or vertical "gradient bars". Each graphical gradient bar 522-526 is related to a different metric, and represents a rate of change of the overall cost with changes to a metric. As noted above, this rate of change is referred to as a cost function gradient for the metric in question.

In the present example, the length of each of the gradient bars 522-526 is related to a cost function gradient. For example, the length of each gradient bar may be proportional or inversely proportional to the value of the cost function gradient. Alternatively, the length of each gradient bar may be a non-linear function of the cost function gradient, such as an exponential or logarithmic function.

In this example, the gradient bar 526 is related to the metric of "Dose-at-Volume for the tumour". So, the rate of change of the cost with respect to changes of the Dose-at-Volume for the tumour is reflected in the length of the gradient bar 526. The gradient bar 524 is related to the metric "Volume-at-dose for a first OAR". So, the rate of change of the cost with respect to the Volume-at-dose for the first OAR is reflected in the length of the gradient bar 524. The gradient bar 522 is related to the metric "Dose-at-Volume for a second OAR". So, the rate of change of the cost with respect to the Dose-at-Volume for the second OAR is reflected in the length of the gradient bar 522.

While the display of the gradient bars is in itself useful to assist the human planner in perturbing the cost function, it may be useful to provide this information in the context of the dose distribution associated with different anatomical structures. Figure 5 illustrates the display of Dose Volume Histograms (DVHs) on the graphical user interface, the DVHs associated with different anatomical structures. Each gradient bar is overlaid on an appropriate DVH.

In the Example of Figure 5, DVH 506 relates to the dose distribution of a tumour, DVH 504 relates to the dose distribution of the first OAR and DVH 502 relates to the dose distribution of the second OAR. These are the dose distributions of a treatment plan optimised by the cost function.

The clinical goals or objectives may also be displayed on the graphical user interface. For example, cross 536 is the desired Dose-at-Volume for the tumour, cross 534 is the desired Volume-at-Dose for the first OAR, and cross 532 is the desired Dose-at-Volume for the second OAR.

Lines representing each plan metric may also be displayed on the graphical user interface. For example, the line passing through the clinical goal 536 represents the metric associated with the clinical goal 536, that is, Dose-at-Volume for the tumour. The point at which this line crosses the DVH 506 is the tumour's Dose-at-Volume achieved by the treatment plan. The difference between this point and the clinical goal 536 represents the deviation of the plan metric from the clinical goal, and contributes to the cost of the cost function accordingly.

The point at which the gradient bars are overlaid on the DVHs is the point at the DVH indicating the achieved metric for the treatment plan. This corresponds to the point at which the line representing the metric crosses the corresponding DVH.

For example, the length of each of the gradient bars 522-526 may be inversely proportional to the value of the cost function gradient. In this example, the longer the gradient bar, the less sensitive the cost is to changes in the metric. Conversely, the shorter the gradient bar, the more sensitive the cost is to changes in the metric. Thus, by viewing the graphical user interface shown in Figure 5, the human planner can see that the cost is not very sensitive to changing the Dose-at-Volume of the second OAR (this cost sensitivity is depicted by gradient bar 522). The cost is more sensitive to changing the Volume-at-dose of the first OAR (this cost sensitivity is depicted by gradient bar 524) and is most sensitive to changing the Dose-at-Volume of the tumour (this cost sensitivity is depicted by gradient bar 526).

In the example of Figure 5, the gradient bars are horizontally oriented when their value relates to dose (e.g., Dose-at-Volume). This allows easy comparison with the x-axis of the DVH. The gradient bars are vertically oriented when their value relates to volume (e.g., Volume-at-Dose). This allows easy comparison with the y-axis of the DVH. In general, the gradient bars are oriented in accordance with the units that they represent and in accordance with the units of the respective axes of the displayed dose distribution.

In addition to the gradient bars, the graphical user interface may also display an arrow at an end of each gradient bar indicating the direction in which the metric will change in response to a change in the cost function.

Using some or all of the above-described information displayed in the graphical user interface, the human planner may perturb the cost function. For example, the planner may choose to change one or more of the clinical goals of the cost function. For instance, the human planner may choose to make the Dose-at-Volume for the tumour less ambitious. Perturbing the cost function will result in the following:
- The DVHs of all three structures will change - that is, the DVHs of the tumour, first OAR and second OAR will all change when the Dose-at-Volume goal for the tumour is made to be less ambitious. For example, improving a DVH for a tumour will generally result in deteriorating a DVH for an OAR.
- The metrics achieved by the treatment plan will also change; that is, the Dose-at-Volume for the tumour may reduce; the Volume-at-Dose for the first OAR may increase and the Dose-at-Volume for the second OAR may decrease.
- The cost function gradients for each metric will change when the cost function is perturbed. For example, the rate of change of the cost with respect to changes of the Dose-at-Volume for the tumour will change, the rate of change of the cost with respect to changes of the Volume-at-Dose for the first OAR will change, and the rate of change of the cost with respect to changes of the Dose-at-Volume for the second OAR will also change.
- The length of the gradient bars also changes in accordance with the changes in the corresponding cost function gradients.

After perturbing the cost function, the display of the DVHs and the gradient bars is updated to reflect the changed DVHs and the changed cost function gradients. The human planner may then use the updated display to inform any decision to further perturb the cost function.

Any further perturbation of the cost function will result in further changes to the DVHs, the achieved plan metrics, the cost function gradients and the gradient bars, and this process iteratively continues until the human planner is satisfied with the cost function and the achieved dose distribution of the cost function.

### Method

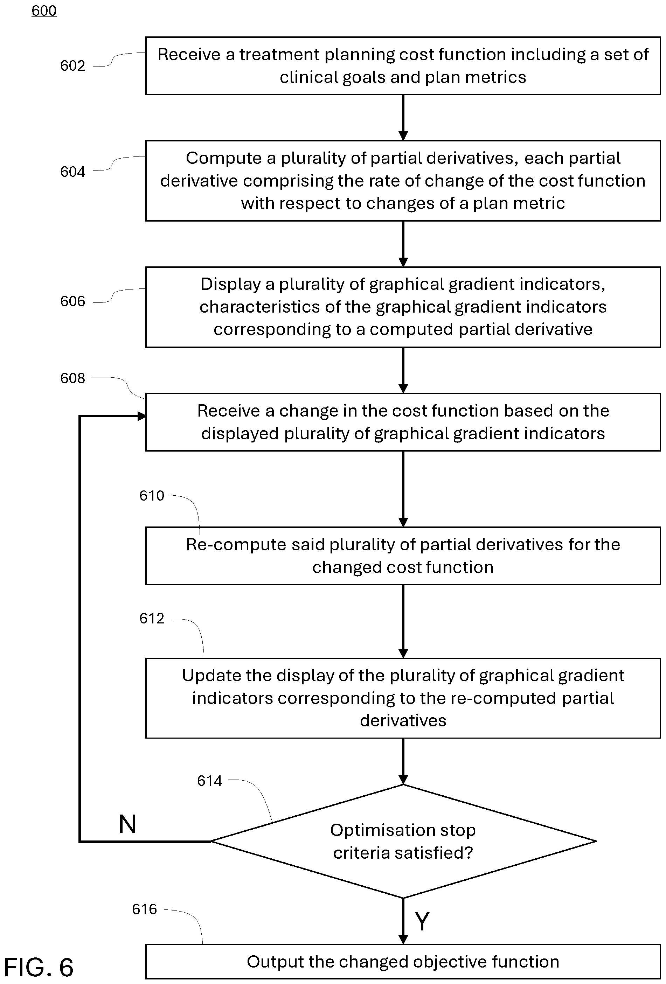

Figure 6 comprises a flowchart of the method of the present disclosure.

In step 602, an initial treatment planning cost function is provided, wherein the cost function comprises a set of clinical goals and plan metrics. The clinical goals and objectives of this initial cost function may be determined using any prior art method, including human trial and error.

In step 604, the rate of change of the cost of the cost function with respect to each plan metric is calculated. These are referred to as cost function gradients. While the cost function includes a plurality of plan metrics, each cost function gradient relates to the rate of change of the cost with only one plan metric. For this reason, the cost function gradient is best referred to as a partial derivative of the cost function with respect to an individual plan metric.

In step 606, the graphical gradient indicators are displayed. At least one characteristic of each graphical gradient indicator is related to the value of the corresponding cost function gradient. For example, the length of the graphical gradient indicator may be proportional to the cost function gradient, and in this case the graphical gradient indicator is referred to as a gradient bar. Optionally, in addition to displaying the gradient bars, the graphical user interface may also display dose distributions such as DVHs of the tumours or OARs related to the plan metrics of the respective cost function gradients. The gradient bars may be overlaid on the DVHs as described above.

In addition to displaying the gradient bars and the DVHs, the clinical goals may also be displayed on the graphical user interface. For example, a Dose-at-Volume goal may be indicated by a cross on the DVH. Also, lines representing each plan metric may be displayed on the graphical user interface, as described above.

In step 608, the human planner may use all of the information displayed in the graphical user interface to make a decision on how to perturb the cost function. For example, goals that are so ambitious that they disproportionately affect the overall cost at the expense of other goals, may be made less ambitious or may be made to be of a lower priority; such goals may be identified by the relative length of the corresponding gradient bar. As a further example, goals that hardly affect the overall cost may be made more ambitious; such goals are identified by the relative length of the corresponding gradient bar.

Also, where the overall cost is highly sensitive to changes of a particular metric, this is identified by the length of the gradient bar. Where the overall cost is not sensitive to changes of a particular metric, this is also identified by the length of the gradient bar. This information can then be used to perturb the cost function to obtain a cost function capable of producing superior treatment plans.

In some examples, instead of the human planner perturbing the cost function, a suitably programmed computer system may perturb the cost function in dependence on the rates of change of the cost in response to changes of the plan metrics.

In step 610, after the cost function is perturbed in the above manner, the partial derivatives of the perturbed cost function with respect to the plan metrics are re-computed. As described above, after the cost function is perturbed, the DVHs of all the structures change, the (dose) metrics achieved for each structure changes, the cost function gradients for each metric changes and the characteristics of the graphical gradient indicators (e.g. the lengths of the gradient bars) may also change.

In step 612, the graphical user interface display is updated. In particular, the updated DVHs and the updated graphical gradient indicators replace the previous DVHs and the previous graphical gradient indicators.

In step 614, it is determined whether certain optimisation stop criteria are satisfied. For example, the human planner may be satisfied with the balance achieved in conflicting clinical goals of the cost function. Alternatively, the iterative process may be stopped after a certain number of iterations.

In step 616, the perturbed cost function is outputted. This perturbed cost function may be used to generate or optimise a radiotherapy treatment plan. For example, the perturbed cost function may be able to provide superior plans than plans provided by the initial cost function.

In summary, the present disclosure provides an ongoing human-computer interactive process that may be used to iteratively determine a radiotherapy treatment plan objective function. This is achieved by displaying graphical gradient indicators that represent the rate of change of the cost of an objective function in response to changes in treatment plan metrics. This information may then be used by a human planner (or a computer) in making decisions regarding how to perturb and evolve the objective function. The display is updated after each perturbation of the objective function and the iterative process continues until a satisfactory objective function is obtained.

Those skilled in the art will recognise that a wide variety of modifications, alterations, and combinations can be made with respect to the above-described embodiments without departing from the scope of the invention, and that such modifications, alterations, and combinations are to be viewed as being within the ambit of the inventive concept.

## Claims

1. A radiotherapy treatment plan generation apparatus configured to:
(i) receive a treatment planning objective function comprising a plurality of clinical goals and plan metrics;
(ii) display one or more graphical gradient indicators, each of the graphical gradient indicators corresponding to a plan metric; wherein a visual characteristic of each of the graphical gradient indicators is related to a rate of change of an objective function value with respect to the plan metric;
(iii) receive input to perturb the objective function based on the displayed one or more graphical gradient indicators;
(iv) update the visual characteristics of the displayed graphical gradient indicators with the rates of change of a value of the perturbed objective function with respect to the plan metrics;
(v) repeat steps (iii)-(iv) until an iteration stop criterion is met;
when the iteration stop criterion is met, output the perturbed objective function.

2. **The** apparatus of claim 1, wherein the graphical gradient indicators each comprise a variable-length shape, and
wherein said visual characteristic comprises the length of the variable-length shape.

3. **The** apparatus of claim 2 wherein the length of each variable-length shape is proportional to the rate of change of the objective function value in response to changes of the plan metric.

4. **The** apparatus of claim 2 wherein the length of each variable-length shape is inversely proportional to the rate of change of the objective function value in response to changes of the plan metric.

5. **The** apparatus of any preceding claim, wherein perturbing the objective function comprises changing aspects of the objective function, such as clinical goals, clinical goal weights or clinical goal priorities.

6. **The** apparatus of any of claims 2 to 5, further configured to perturb the objective function based on the length of the variable-length shapes.

7. **The** apparatus of any preceding claim, further configured to scale the graphical gradient indicators with a linear scaling coefficient.

8. **The** apparatus of claim 7, further configured to use (i) a relatively smaller linear coefficient to visualise rates of change with respect to a current treatment plan solution, and (ii) a relatively larger linear coefficient to visualise differences between the rates of change.

9. **The** apparatus of any preceding claim, further configured to scale the graphical gradient indicators with a non-linear scaling coefficient, such as an exponential coefficient, or a logarithmic coefficient.

10. **The** apparatus of any preceding claim, wherein one or more of the graphical gradient indicators each comprise a coloured object, and wherein said visual characteristic comprises a colour of the object.

11. **The** apparatus of any preceding claim, further configured to display an arrow indicating the direction of change of the plan metric in response to perturbing the objective function.

12. **The** apparatus of any preceding claim, further configured to display a dose distribution related to the objective function and overlay the graphical gradient indicators on the displayed dose distribution.

13. **The** apparatus of claim 12, wherein the dose distribution comprises a dose-volume histogram.

14. **The** apparatus of claim 12 or claim 13, wherein the displayed dose distribution relates to a region of interest, such as an organ at risk or a tumour.

15. The apparatus of any preceding claim wherein the rates of change of the objective function value in response to changes to the plan metric comprises a partial derivative such as: $G = \frac{{λ}_{G}}{{\partial C}_{\overline{\partial m}}}$ where:
G = value of the graphical gradient indicator
λ_{G} = scaling coefficient
C = objective function value
m = plan metric
